(19) Europäisches Patentamt
European Patent Office
Office européen des brevets

(11) EP 2 878 304 A1

(12) EUROPEAN PATENT APPLICATION

(43) Date of publication:
**03.06.2015 Bulletin 2015/23**

(51) Int Cl.:
***A61K 36/28*** *(2006.01)*

(21) Application number: **14195927.0**

(22) Date of filing: **02.12.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **02.12.2013 IT RM20130652**

(71) Applicant: **Aboca S.p.A. Societa' Agricola**
**52037 Sansepolcro (AR) (IT)**

(72) Inventors:
- **Mercati, Valentino**
  **52037 Sansepolcro AR (IT)**
- **Mattoli, Luisa**
  **52037 Sansepolcro AR (IT)**

(74) Representative: **Predazzi, Valentina et al**
**Società Italiana Brevetti S.p.A.**
**Piazza di Pietra, 39**
**00186 Roma (IT)**

(54) **New fractions of chamomile extracts and uses thereof**

(57) The present invention relates to new fractions of chamomile extract wherein only traces of total apigenins and essential oil are present, a process for the preparation of such extract, compositions comprising it, and the use of the extract and of the compositions for the protection of the skin or mucous membranes.

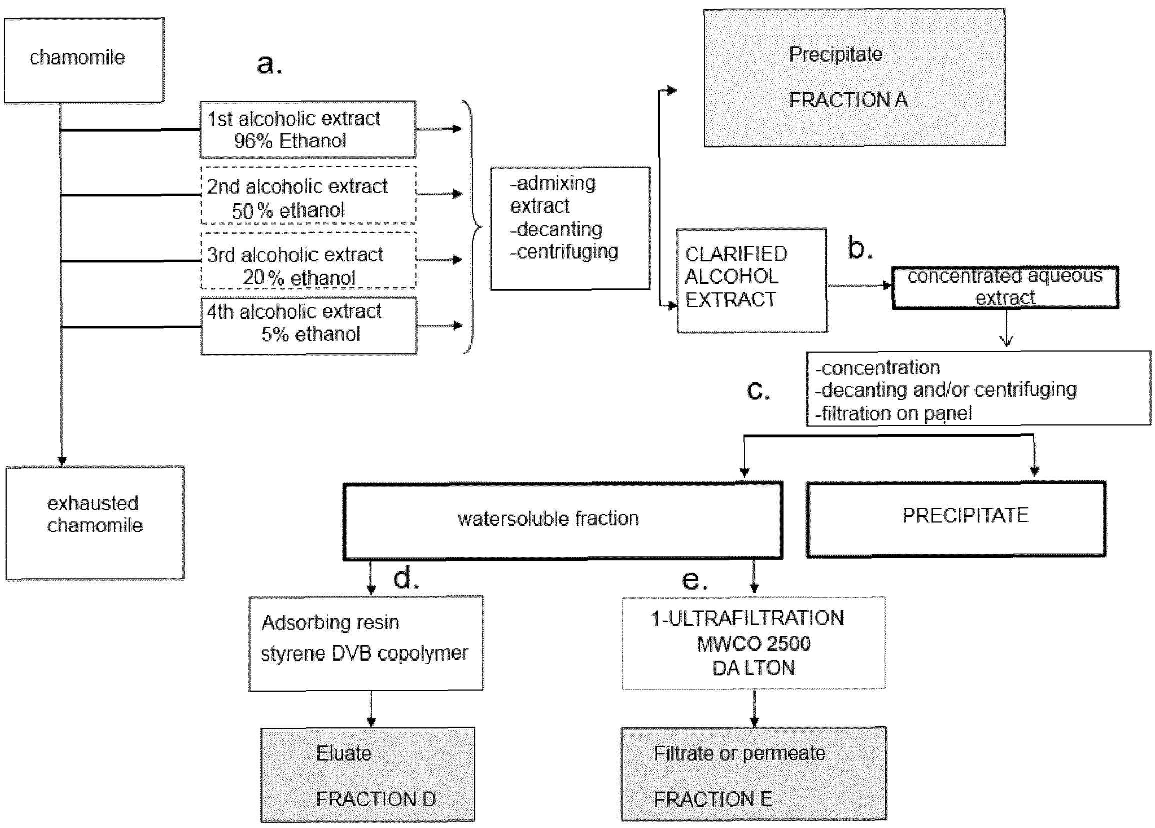


Fig. 1



Printed by Jouve, 75001 PARIS (FR)

## Description

TECHNICAL FIELD OF THE INVENTION

The present invention relates to new fractions of chamomile extract in which only traces of total apigenins are present, a method for the preparation of said fractions, compositions comprising them, and the use of the fractions and of the compositions for the protection and/or the repair of the skin or mucous membranes.

STATE OF THE PRIOR ART

By "common chamomile" it is meant *Matricaria recutita, Matricaria chamomilla* or *Chamomilla recutita,* which is an annual fragrant herb high up to 0.6 m, with flower heads about 2 cm large. The drug consists of dried flowers, where by "flower" it is meant flower heads with involucre, comprising 12-17 bracts, white ligulate flowers and yellow tubulate flowers and flowers with 1-3 cm of stalk (Official Italian Pharmacopoeia).

By "flowers" therefore are meant flower heads with involucre comprising 12-17 bracts, white ligulate flowers and yellow tubulate flowers and flowers with 1-3 cm of stalk.

The main constituent of *Matricaria* flowers is the essential oil with particular components giving pharmacological properties to common chamomile. The oil possesses bactericidal and antifungal activities, in particular against Gram-positive bacteria.

Moreover, chamomile flavones exhibit local antiinflammatory activity; in particular, apigenin is able to penetrate the deeper strata of the skin (Herbal Drugs and Phytopharmaceuticals).

As reported in the ESCOP monograph, chamomile is indicated for internal use in the symptomatic treatment of intestinal disorders, and for external use in case of minor irritations and inflammations of the skin and mucous membranes.

PHYTOCHEMICAL COMPOSITION: The main constituents of common chamomile are essential oil (0.5-1.5%), as reported in the ESCOP monograph, flavonoids, among which apigenin, apigetrin, rutin, luteolin and quercimetrin.

The essential oil consists for about 50% of sesquiterpenes, such as (-)$\alpha$-bisabolol and its oxides A B and C, of about 25% of cis- and trans-en-in-dicycloethers (or spiroethers) and matricin (up to 15%), a chamazulene precursor converted during distillation. Other components of chamomile flowers are: coumarins, such as umbelliferone and its methylether herniarin, phenolic acids and polysaccharides.

Patent document EP 0096016 describes a lipophilic fraction of chamomile with strong antibacterial properties and a process for the preparation thereof. According to what is described in the document, the extract would be free from volatile components, apigenins included, even though in the same document the Applicants state that the constituents of the lipophilic fraction have been identified.

Patent document US 2009/285921 describes an aqueous extract of chamomile for the treatment of proliferative and/or viral pathological conditions.

The extract described, which would have antiviral and antitumour activities, is prepared with methods comprising thermal treatments, filtrations and treatments with lipophilic substances for decontamination from endotoxins and subsequent microfiltrations.

Patent document WO 2009/138860 describes extracts of tubular flowers (yellow flowers) of chamomile that would have activities of reduction of DNA- and RNA synthesis peculiar to the aqueous extract.

Let us mention that chamomile yellow flowers contain total apigenins (0.01-0.2%), even though having them with a lower content compared to white flowers and to the whole flower as defined above (about 0.2-0.5%).

Therefore, the state of the art provides some particular fractions of chamomile flowers or of sole chamomile yellow flowers for use as antibacterial, antiviral or antiproliferative agents. Therefore, ways to use chamomile extracts exerting protection effects of the skin or the mucous membranes on the basis of mechanical-type properties, and not of pharmacological-type ones, are not described in the literature.

SUMMARY OF THE INVENTION

The Authors of the present invention have discovered that it is possible to isolate fractions from chamomile flower heads which maintain the mucoadhesive properties of the reference hydroalcoholic extract but are substantially depleted in total apigenins. The present invention relates to selected fractions of chamomile flower heads extract substantially depleted in the known pharmacologically active components mainly represented by total apigenins, of which only traces remain, to compositions comprising such fractions and to processes for obtaining them. The fractions according to the invention may be used for the protection and/or the repair of the mucous membranes or of the skin, or to cooperate with such protection and/or repair. Among the interesting features of such fractions there is the ability to retain desired substances on the mucous membranes by virtue of their mucoadhesive properties. The fractions of the invention

therefore provide a new source of material of plant origin useful for the protection and/or the repair of the mucous membranes and of the skin, and/or also as excipient/carrier capable of retaining for a longer time desired substances on the mucous membranes, wherein the pharmacological effects attributable to the components known as active ingredients (principles), are substantially reduced or even eliminated.

Said condition is particularly desirable when materials of natural or plant origin are to be used for actions of a mainly mechanical type (i.e., not for pharmacological actions having, for instance, a direct effect on the activation of receptorial, immune pathways or metabolic processes). The use of substances having a mainly mechanical effect, and therefore not pharmacological, immune, metabolic effect, such as, e.g. substances having a mucoadhesive effect, is desirable, for instance, to cooperate with a pharmacological activity. By minimizing the presence of substances with pharmacological activity in the material of natural or plant origin as in the present invention, it is obtained a material with a mainly mechanical effect that can be used in combination with drugs, minimizing possible undesired interactions with drugs, and at the same time exploiting the typical feature of substances of plant origin of having a vast number of components capable of exerting a protective effect on skin and mucous membranes, besides the pharmacologically active ones commonly used.

The use of fractions of plant or natural extracts depleted in the most known pharmacologically active ingredients, and enriched in the remaining substances, entails the dual advantage of providing a material with a mainly mechanical protective action (mucous membrane protection or skin protection) and of enabling a concrete and useful use of materials normally discarded in extraction processes of plant or natural active ingredients.

The present invention therefore relates to fractions of chamomile flower heads extracts depleted in total apigenins, with a total apigenins concentration, expressed as percentage by weight (weight%) of the lyophilised fraction, of ≤0.2%, or even ≤0.08% or even ≤0.001% and optionally depleted also in essential oil; their use as in the protection of the skin or mucous membranes and/or as carriers with a high mucoadhesive ability; compositions comprising such fractions; their use in the protection of the skin or mucous membranes and/or for the carrying of desired active ingredients on the mucous membranes and processes for the preparation of such fractions and such compositions.

The invention also relates to a process for the preparation of fractions of chamomile flower heads extract as defined above, comprising the following step:

a. preparing a hydroalcoholic extract of chamomile flower heads and subjecting said extract to centrifugation and/or decantation, thereby obtaining a precipitated fraction A and a clarified alcoholic extract, and collecting said precipitated fraction A characterised by a concentration of total apigenins, expressed as percentage by weight, of ≤0.08.

The process may also comprise further steps

b. obtaining from the clarified alcoholic extract prepared in a. a concentrated aqueous extract,
c. decanting and/or centrifuging and filtering said aqueous extract, thereby collecting a water-soluble fraction,
d. subjecting said water-soluble fraction obtained in c to a step (passage) on adsorbing resin, thereby collecting a fraction D, corresponding to the eluate obtained from said passage on resin, characterised by a total apigenins concentration of ≤0.001% and an essential oil concentration of ≤0.01% expressed as weight%;
or it could comprise the above-described steps a. b. c. and step
e. subjecting the water-soluble fraction obtained in c. to ultrafiltration on a membrane and collecting a fraction E, corresponding to the permeate obtained from said ultrafiltration, characterised by a concentration of total apigenins of between 0.2% and 0.09% and of essential oil of ≤0.01% expressed as weight%.

The invention also relates to the preparation of said fractions A, D and/or E, or the preparation of mixtures of said fractions A and/or D and/or E with one or more natural active ingredients (principles) and/or extracts deriving from sources different from chamomile, or anyhow natural active ingredients that be other than apigenins. The invention further relates to the preparation of pharmaceutical compositions or of foods for special medical purposes (or medical foods) containing one or more of said fractions or mixtures in combination with other natural substances and/or active ingredients deriving from sources different from chamomile, or anyhow natural active ingredients that be other than apigenins. Furthermore, the invention relates to the use of said fractions alone or mixed with each other and/or with said active ingredients or other pharmacologically inert components, or the use of said compositions for the protection of the mucous membranes and of the skin. Moreover, the invention relates to the use of said fractions or mixtures as carriers capable of retaining said active ingredients (deriving from sources different from chamomile, or anyhow from natural active ingredients that be other than apigenins) on the mucous membranes by virtue of their effective mucoadhesive properties.

The fractions of the invention could optionally be further depleted in chamomile essential oil.

The invention therefore generally relates to fractions of chamomile extracts (or mixtures thereof or compositions comprising them) and uses thereof in the protection and/or repair of mucous membranes or of the skin; said effect is

due to the mechanical features of mucoadhesion of said fractions, jointly with the substantial absence of the effects attributable to chamomile active ingredients commonly used, i.e. apigenins. Optionally, the fractions or mixtures thereof are also free from the effects attributed to essential oils.

Percentage values are reported with respect to the weight of the fractions object of the invention after drying treatment by lyophilisation, therefore by "expressed as percentage by weight" it is meant "expressed as percentage by weight of the lyophilised fraction" o "expressed as percentage by weight of the lyophilised extract".

DETAILED DESCRIPTION OF THE FIGURES

Figure 1 shows a block diagram of an embodiment of the process of the invention.

Figure 2 shows the result of the comparative test related to the mucoadhesive properties of the fractions of the invention (fractions A, D and E, in comparison with a fraction prepared according to Example 8 of document WO 2009/138860) compared by adhesion assay on an inclined plane.

SHORT GLOSSARY

NMWCO = Nominal Molecular weight Cut-off.

PERMEATE: extract fraction that has passed through the semi-permeable ultrafiltration membrane.

RETENTATE: extract fraction that did not pass through the ultrafiltration membrane or material adsorbed on resin.

ELUATE: material not adsorbed on resin following adsorption passages thereon.

ULTRAFILTRATION: filtration technique on semi-permeable membrane characterised by pores having sizes of 100.000 daltons (0.1 μm) to 500 daltons (about 0.005 μm)

CHAMOMILE PARTS USED: flower heads as defined above.

ADSORBING RESIN: denotes a resin able to adsorb aromatic and/or apolar substances, like, e.g., a high-porosity hydrophobic adsorbing resin. This type of resin consists of microspheres of a diameter of 0.2 mm - 0.08 mm, with an uniformity coefficient < 1.5 obtained by polymerization of Styrene and DVB without active groups, characterised by a highly porous physical structure having the parameter relative to the pore volume equal to about 1.3 ml/g, enabling adsorption and selective elution of organic substances, preferably of aromatic nature. A non-binding example of resin according to the invention is amberlite XAD-2, serdolit PAD-II, ADS TQ 318 or resins similar thereto.

By "NOT GREATER THAN", relative to the amounts of active ingredients in the extract, in the present description it is meant ≤.

TOTAL APIGENINS: in the present description the indicated concentrations of total apigenins are quantitatively determined as percentage by weight of the sample lyophilised by HPLC chromatographic method.

ESSENTIAL OIL: in the present invention, the indicated concentrations of essential oil are quantitatively determined as percentage by weight of the sample lyophilised by GC-MS gas-chromatographic method.

DETAILED DESCRIPTION OF THE INVENTION

As indicated above, in the present description there are provided fractions of chamomile flower heads extract depleted in total apigenins, with a total apigenins concentration expressed as percentage by weight of between 0.2% and 0.09%, or even ≤0.08% or even ≤0.001%, wherein said fractions have good mucoadhesive properties.

Mucoadhesive properties were assayed by two methods described in detail in the experimental section, and briefly summarized below.

A first method of measuring is given by the evaluation of the mucoadhesive ability of the sample by measuring its ability to bind to mucin disposed on an inclined plane, allowing the moving away by sliding of the sample without mucoadhesive ability. The second method enables to evaluate the mucoadhesive ability of the sample by inhibition of the lectin/glycoprotein bond expressed on the cell membrane of cells coming from a mucous membrane.

By applying the fractionation process described below, it was possible to obtain fractions of chamomile extract depleted in total apigenins and concomitantly enriched in the remaining substances, which exhibited an effective protective action on skin and on mucous membranes, as evident from the mucoadhesive assays carried out.

Compared to commonly used chamomile fluid extracts, soft extracts, lyophilised or dry extracts, which comprise a total apigenins amount equal to about 0.5% by weight, the fractions of the present invention comprise a total apigenins amount expressed as percentage by weight of between 0.2% and 0.09% or even of ≤0.08% or even of ≤0.001 %.

Moreover, in some embodiments the fractions of the invention are also depleted in chamomile essential oil, exhibiting a concentration of chamomile essential oil, expressed as percentage by weight, of ≤0.01%.

In particular, the fractions of the invention could be a fraction with a concentration of total apigenins expressed as percentage by weight of between 0.2% and 0.09%, a fraction with a concentration of total apigenins expressed as percentage by weight of ≤0.08% and a fraction with a concentration of total apigenins expressed as percentage by weight of ≤0.001%.

Said fractions can be solely hydrophilic fractions or mixed hydrophylic/lipophylic fractions.

In one embodiment, the fraction according to the invention could be a fraction with a concentration of total apigenins expressed as percentage by weight of between 0.2% and 0.09% and a concentration of chamomile essential oil, expressed as percentage by weight, of ≤ 0.01%.

In a preferred embodiment, said fraction is hydrophilic.

In another embodiment, the fraction according to the invention could be a fraction with a concentration of total apigenins expressed as percentage by weight of ≤0.001% and a concentration of chamomile essential oil expressed as percentage by weight ≤ 0.01%.

In a preferred embodiment, said fraction is hydrophilic.

In yet another embodiment, the fraction according to the invention could be a fraction with a concentration of total apigenins expressed as percentage by weight comprised and 0.08% and a concentration of chamomile essential oil expressed as percentage by weight of ≤ 0.35%.

In a preferred embodiment, said fraction is a mixed hydrophilic/lipophilic fraction.

The fractions according to the present description exhibit surprising mucoadhesive activities, in some cases even greater than the total extract used as control.

Evidently, the values of total apigenins present in the fractions of the invention correspond in practice to traces of total apigenins. Such values cannot be held accountable for the mucoadhesive properties exhibited by the fractions of the invention and cannot induce the pharmacological activities for which chamomile and chamomile extracts are commonly used.

The selected fractions according to the present description, substantially depleted in active ingredients (principles) like total apigenins, surprisingly preserve or even improve mucoadhesive properties with respect to the non-fractionated hydroalcoholic chamomile extract used as reference, as can be seen in Table 1 reported below.

The table below shows the mucoadhesive properties (evaluated by cell adhesion method explained in greater detail in the experimental section below) of the fractions with respect to a total lyophilised chamomile extract used as control.

**Table 1**

| ANALYZED SAMPLE | Essential oil concentration, expressed as % by weight | Total apigenins concentration, expressed as % by weight | Cell adhesion assay-% | water-soluble molecules with MW of > 25000 Da% |
|---|---|---|---|---|
| REFERENCE CHAMOMILE EXTRACT, LYOPHILISED, | 0.2 | 0.5 | 63 | 19.08 |
| CHAMOMILE, FRACTION A, LYOPHILISED mixed hydrophilic/lipophilic fraction | 0.35 | 0.064 | 69 | 12.88 |
| CHAMOMILE, FRACTION D, RESIN-ELUTED FRACTION, LYOPHILISED hydrophilic fraction | <0.01 | 0.001 | 30 | 28.66 |
| CHAMOMILE, FRACTION E, PERMEATE, GH<2500 DA, LYOPHILISED hydrophilic fraction | <0.01 | 0.18 | 40 | 21.24 |

As can be seen from the Table, mucoadhesive abilities seem to be totally independent of the concentration of the active ingredients. Unpublished data enabled to single out numerous fractions obtainable in the various stages of extraction processes in which the mucoadhesive properties are practically absent or anyhow lower than 30% in the cell adhesion assay used.

The depletion in the most known active ingredients of chamomile, represented by total apigenins, is directly measurable by the measurement of percentage by weight of total apigenins in HPLC.

The measurement of total apigenins as reported in the present description is obtainable with standard processes by HPLC chromatographic method.
Said method of analysis provides a double extraction of the sample under examination with a 40% ethanol solution, by ultrasounds at a temperature of 35°C ±3°C; and HPLC analysis by C18 reverse-phase column and UV detector at a wavelength of 335 nm.

Also the depletion with regard to the content of essential oil is directly measurable by evaluation of the percentage area of the main sesquiterpene constituents in GC-MS. Said method provides the extraction, under vapour stream, of essential oil from chamomile flowers and its use as reference standard of pure essential oil of chamomile. Then, it provides the extraction of essential oil from analyzed samples by hexane, and quantitative analysis by gas chromatography and single-quadripole mass analyzer provided with an electronic impact source.

The mucoadhesive properties are evaluated by a cell adhesion assay described below. Evidently, such properties can also be evaluated with further techniques known to a person skilled in the art.

In the reference extract, total apigenins are represented by about 0.3063% of apigenin-7-O-glucoside, about 0.1475% of apigenin-7-O-(6-acetyl)-glucoside and about 0.0360% of free apigenin.

In particular, fraction A of the invention is a mixed hydrophilic/lipophilic fraction and has a concentration of total apigenins expressed as percentage by weight ≤0.08% (i.e., of 0.064%) markedly reduced with respect to the starting extract, resulting in a depletion in total apigenins greater than 80% with respect to the starting extract. This fraction has a mucoadhesion, expressed as cell adhesion percentage calculated with the process described below, equal to about 69%. Fraction A is therefore depleted of about 83% in the active ingredient total apigenins with respect to the reference chamomile extract, represented by the lyophilisate of the extract in 40% alcohol and exhibits mucoadhesive properties, in cell adhesion assay, even greater than the reference extract.

In particular, in fraction A, total apigenins are represented by about 0.0302% of apigenin-7-O-glucoside, about 0.0214% of apigenin-7-O-(6-acetyl)-glucoside and about 0.0125% of free apigenin.

Fraction D of the invention exhibits a concentration of total apigenins of ≤0.001% by weight and of essential oil of ≤0.01% by weight, and therefore practically nullified with respect to the starting extract (N.B. in the measurement assays apigenins, as well as essential oil, were not even detectable), resulting in a depletion in total apigenins and essential oil greater than 99% with respect to the starting extract. Notwithstanding the very marked depletion carried out by passage on (through) resin, this fraction retains anyhow a mucoadhesion expressed as cell adhesion percentage, calculated with the process described below, equal to about 30%. Therefore, fraction D is depleted of >99% in the active ingredient total apigenins and in essential oil with respect to the reference chamomile extract, represented by the extract lyophilisate in 40% alcohol and has a barrier effect still appreciable, notwithstanding the marked depletion in components caused by passage on resin.

In particular, in fraction D the total apigenins are represented by about 0.000 % of each apigenin reported above in detail.

Fraction E of the invention exhibits a concentration of total apigenins of ≤0.2% by weight, in particular exhibits a concentration of apigenins expressed as percentage by weight of between 0.2% and 0.09%, markedly reduced with respect to the starting extract, and essential oil ≤0.01% by weight, resulting in a depletion in total apigenins greater than 55% with respect to the starting extract and a depletion in essential oil greater than 99% with respect to the starting extract. This fraction has a mucoadhesion, expressed as cell adhesion percentage calculated with the process described below, equal to about 40%. Fraction E is therefore depleted of about 58% in the total apigenins active ingredient and of more than 99% with respect to the reference chamomile extract, represented by the extract lyophilisate in 40% alcohol, and however exhibits mucoadhesion abilities that are still appreciable.

In particular, in fraction E the total apigenins are represented by about 0.1211% of apigenin-7-O-glucoside, about 0.0491% of apigenin-7-O-(6-acetyl)-glucoside and about 0.0114% of free apigenin.

According to the above-described features of fractions D and E, the fractions of the invention, besides being depleted in total apigenins, are also depleted in essential oil up to >99% by weight, and exhibit mucoadhesion abilities still appreciable with respect to the reference chamomile extract, represented by the extract lyophilisate in 40% alcohol which did not undergo fractionation.

The Inventors of the present invention have selected the above-mentioned fractions with respect to other fractions obtainable with the process as described in the present document, as they were the only ones exhibiting sufficient reductions in total apigenins and improving (despite at times having similar compositions) or maintaining appreciable mucoadhesive abilities with respect to a reference extract represented by the chamomile extract lyophilisate in 40% alcohol.

Many of the fractions obtained, in fact, besides exhibiting an enrichment in chamomile active ingredients, (marked pharmacological effect), in some cases exhibited no mucoadhesive effect. Moreover, as shown by data in Figure 2 (Table 4, Example 6), the fractions of the present invention also have greater mucoadhesive abilities with respect to fractions reported in the literature.

Therefore, evidently it is difficult to foresee whether and which fractions of a plant extract be capable of exhibiting

mucoadhesive abilities.

The fractions of the invention, represented by fractions A, D and/or E or by mixtures thereof, are therefore useful for their mucoadhesion properties in all those cases in which a protection of skin or mucous membranes is needed or desirable. This may occur in those cases in which a medicament or an agent taking advantage from adhesion to the skin or to the mucous membranes has to be administered, cases in which the fractions have the object of cooperating with the action of the medicament not only by effects of physical protection due to the fact that they adhere to the mucous membrane or the skin, but also by acting as carriers enabling a greater adhesion of the medicament itself to the concerned zones. The fractions or mixtures thereof are therefore extremely useful in those cases in which the protection of a partially compromised skin or mucous membrane is preferable or desirable so as to enable a better and quicker healing thereof, enabling a greater adhesion to the sites of interest of the medicaments or of protective agents administered; in those cases in which an individual has a chronic disorder in which skin or mucous membranes undergo irritations or alterations whereby a mucoadhesion effect can prolong the duration of the active ingredients and/or of desired protective components on the skin or on the mucous membrane, and therefore in all those cases in which it is desired to carry particular substances in a preferential way and with a prolonged duration on the mucous membranes or on the skin.

By "protection of the mucous membranes" it is meant a mechanical protection, given by the mucoadhesive abilities of the fractions of the invention or of mixtures thereof, which could be associated with a pharmacological protection related to active ingredients different from apigenins mixed with the fractions of the invention. In the present description said mucous membranes can be the oral mucosa, gastric mucosa, intestinal mucosa, the nasal mucosa, the vaginal mucosa, the uterine mucosa, the rectal mucosa.

Fractions A, D and E could be used alone or mixed with each other and could be optionally further mixed (alone or in combination) with extracts of other plants and/or natural active ingredients not extracted from chamomile (or anyhow wherein said active ingredients are not apigenins), as active ingredients of other plants or of other natural products in order to boost the mechanical activity, as defined above, of the resulting mixtures.

According to the invention, the fractions could be obtained by the process described hereinafter.

Process for the preparation of fractions of chamomile extract depleted in total apigenins comprising the following step:

a. preparing a hydroalcoholic extract of chamomile flower heads and subjecting said extract to centrifugation and/or decantation, thereby obtaining a precipitated fraction A and a clarified alcoholic extract and collecting said precipitated fraction A characterised by a concentration of total apigenins, expressed as percentage by weight of the lyophilised fraction, of ≤0.08%.

Fraction A also exhibits a good mucoadhesion, both when calculated by cell adhesion assay and by adhesion assay with mucin on an inclined plane, as evident in Table 1 and in Figure 2 and Table 4, example 6.

The process may comprise further steps:

b. obtaining from the clarified alcoholic extract prepared in a. a concentrated aqueous extract,
c. decanting and/or centrifuging and filtering said aqueous extract, thereby collecting a water-soluble fraction;
d. subjecting said water-soluble fraction obtained in c to a step on adsorbing resin, thereby collecting a fraction D, corresponding to the eluate obtained from said step on resin, characterised by a concentration of total apigenins of ≤0.001% and an essential oil concentration of ≤0.01%, expressed as weight%.

Fraction D also has a good mucoadhesion, both when calculated with cell adhesion assay and with adhesion assay with mucin on an inclined plane, as evident in Table 1 and in Figure 2, and in Table 4, example 6.

Or the process may comprise further steps:

b. obtaining from the clarified alcoholic extract prepared in a. a concentrated aqueous extract,
c. decanting and/or centrifuging and filtering said aqueous extract, thereby collecting a water-soluble fraction:
e. subjecting the water-soluble fraction obtained in c. to ultrafiltration on a membrane and collecting a fraction E, corresponding to the permeate obtained from said ultrafiltration, characterised by a concentration of total apigenins of ≤0.2% and an essential oil concentration of ≤0.01%, expressed as percentage by weight (weight%).

Fraction E also exhibits a good mucoadhesion, both when calculated with cell adhesion assay, and a particularly effective mucoadhesion with adhesion assay with mucin on an inclined plane, as evident in Table 1 and in Figure 2 and Table 4, example 6.

Fraction A, obtainable by the above-described step a., exhibits quite interesting features reported in Table 1 above, i.e.: total apigenins concentration ≤ 0.08%, mucoadhesive properties, assayed by cell adhesion method reported in the experimental section, equal to about 69%.

Fraction D, obtainable by the above-described step d., exhibits very interesting features reported in Table 1 above, i.e.:

total apigenins concentration ≤0.001%, essential oil ≤0.01% mucoadhesive properties, assayed by cell adhesion method reported in the experimental section, equal to about 30%.

Fraction E, obtainable by the above-described step e., has it also very interesting features reported in Table 1 above, i.e.:

total apigenins concentration ≤ 0.2% expressed as weight% or also total apigenins concentration of between 0.2% and 0.09% expressed as weight%, concentration of essential oil ≤0.01% expressed as weight%, mucoadhesive properties, assayed by cell adhesion method reported in the experimental section, equal to about 40%. The process according to the invention could therefore be used to produce one or more of the fractions of interest described herein, A, C or E.

According to one embodiment the invention relates to hydrophilic or mixed hydrophilic/lipophilic fractions of chamomile flower heads obtainable by step a, or by steps a. b. c. followed by steps d. or e., wherein said fractions are characterised by a total apigenins concentration of ≤ 0.2% expressed as weight%.

Said fractions are also characterised by a mucoadhesion percentage measured by mucosal cell adhesion assay as reported in Table 1 and described above and/or by a mucoadhesion percentage measured by mucoadhesion assay with an inclined plane equal to at least 73%.

The percentage of mucoadhesion on an inclined plane of the fractions, like fractions A, D and E, is, in particular, as reported in Figure 2 and Table 4 in Example 6 describing it. In a further embodiment, the invention relates to hydrophilic fractions of chamomile flower heads obtainable by steps a. b. c. followed by step d. or e. of the process as described above, wherein said fractions are characterised by a concentration of total apigenins expressed as percentage by weight of ≤ 0.2%.

Said fractions are also characterised by a concentration of essential oil expressed as percentage by weight of ≤0.01%.

Said fractions are also characterised by a percentage of mucoadhesion, measured by mucosal cell adhesion assay as described below, equal to at least about 30% (or even 40% in case of fraction E) and by a percentage of mucoadhesion, measured by mucoadhesion assay with an inclined plane, equal to at least 75%.

The percentage of mucoadhesion on an inclined plane of fractions D and E is, in particular, as reported in Figure 2 and Table 4 in Example 6 describing it.

The process described herein could be carried out starting from chamomile flowers, both fresh and dried, or alternatively from fluid extracts (like, e.g., hydroalcoholic extracts) or dry chamomile extracts.

The dry extracts could be resuspended/resolubilised by means of hydroalcoholic solutions, e.g. by using a solid/solvent ratio of 1:5, or 1:7 or 1:8 or 1:10, putting under stirring the mass for a time ranging from 4 to 8 hours.

Embodiments for each step of the above-described process are reported in detail below.

Step a. of the process:

a. preparing a hydroalcoholic extract of chamomile flower heads and subjecting said extract to centrifugation and/or decantation, thereby obtaining a clarified alcoholic extract. According to the invention, the above-described process can be carried out by performing at step a. at least two, at least three, or at least four steps in ethanol at decreasing concentrations, wherein the decreasing concentrations of ethanol are from about 96% ethanol to about 0% ethanol.

For instance, step a. of the process can be carried out by performing a step in about 96% ethanol, and a step in about 5% ethanol.

Or, step a. of the process could be carried out by performing a step in about 96% ethanol, a step in about 50% ethanol and a step in about 20% ethanol.

Or, step a. of the process could be carried out by performing a step in about 96% ethanol, a step in about 50% ethanol and/or a step in about 20% ethanol and a step in about 20% ethanol.

The extracts obtained as described above are gathered to form a hydroalcoholic extract. These can be gathered, e.g., in equal mutual ratios, such as 50:50 in case of two steps in ethanol, or such as 33.3:33.3:33.3 in case of three steps in ethanol or 25:25:25:25 in case of four steps in ethanol. Evidently, the extracts obtained by performing the various steps in ethanol described above could be gathered into different ratios according to the general knowledge of the technician in the field.

Fraction A of the invention, obtainable as described above, can be used as is or subjected to a lyophilisation process to provide a lyophilised fraction of extract useful for formulations having therapeutic employ for internal and/or

external use.

As already indicated above, the clarified alcoholic extract (or hydroalcoholic extract) obtained in step a. besides fraction A, could be used in successive steps b. c. and d. or b. c. and e.

Initially, the hydroalcoholic extract obtained in a. will then used to prepare a concentrated aqueous extract in step b.

Said concentrated aqueous extract can be prepared by concentrating the hydroalcoholic extract obtained in a. by alcohol evaporation, then it is decanted and/or centrifuged and/or filtered and the resulting concentrated aqueous phase is collected. For instance, in case of static and/or centrifugal decantation, the aqueous supernatant is collected whereas in case of filtration the filtrate is collected. Alternatively, alcohol evaporation can be performed after the centrifugation and/or decantation and/or filtration steps.

Decantation can be performed, e.g., 1 to 72 hours, and can be followed or replaced by one or more centrifugations to about 3000-4000 (e.g., about 3500) rpm for a period of between 1 and 10 minutes, e.g. about 5 minutes. For the filtration suitable filters known to a technician in the field can be used, like, e.g., panel filters having a cutoff of about 25 micrometers. In some cases, further consecutive filtrations of the extract can be performed on filters having gradually decreasing cutoffs, from 200 micrometers down to 0.1 micrometers.

The supernatant or the filtrate obtained after this step or steps is then collected and subjected, in case this has not already been done before, to alcohol evaporation by standard techniques, like e.g. by use of thin film distillation system, or a batch concentration system according to standard protocols commonly used by the technician in the field.

The result of this evaporation is a concentrated aqueous extract that, in step c. of the process, is in turn subjected to decantation and/or filtration.

As already mentioned, this evaporation can be performed also before the step of decantation and/or centrifugation and/or filtration.

The concentrated aqueous extract (which could be indicated in the text or in the drawings also as concentrated aqueous solution) can be subjected to decantation for a period of between 1 and 72 hours, and the supernatant is then collected, thereby obtaining a clarified or clear solution.

Decantation can be followed or replaced by one or more centrifugations to about 3000-4000 (e.g., about 3500) rpm for a period of between 1 and 10 minutes, e.g. about 5 minutes.

Alternatively, the concentrated aqueous extract is filtered, thereby obtaining a clarified solution. In some cases, further consecutive filtrations of the extract can be performed on filters having gradually decreasing cutoffs, starting from 200 micrometers until arriving to 0.1 micrometers. The filtration can be performed, for instance, on a panel filter having a cutoff of 25 micrometers.

In a further embodiment the concentrated aqueous extract can be decanted as described above and the supernatant obtained can then be filtered as described above.

The supernatant or the filtrate obtained as described above, depleted in essential oil, are recovered and subjected to a further step of depletion in total apigenins by ultrafiltration or passage on adsorbing resin.

The method of the invention can therefore comprise a further step, described below as d. or e., of depletion of undesired substances from the supernatant or the filtrate obtained in step c. described above before collection of the desired fraction, wherein said supernatant or filtrate is subjected to one or more steps of depletion in total apigenins.

This further step enables to obtain further fractions of chamomile extract comprising total apigenins at a concentration expressed as percentage by weight (weight%) of $\leq$0.001% in case step d. is performed, or comprising total apigenins at a concentration expressed as percentage by weight of $\leq$0.2% (of between 0.2% and 0.09%) in case step e is performed.

This step could be carried out by passage through adsorbing resin of the supernatant or filtrate obtained in c. (step d.) or by ultrafiltration of the supernatant or filtrate obtained in c. (step e.).

In case ultrafiltration is performed, the supernatant or filtrate obtained in c. is subjected in e. to one or more ultrafiltration steps and the permeate (fraction E) is recovered, wherein said permeate comprises total apigenins at a concentration of between 0.2% and 0.09% expressed as weight% and a chamomile essential oil concentration of $\leq$0.01% expressed as weight%.

In step e., the aqueous extract (result of the filtration or supernatant of the decantation in c.) is subjected to one or more steps of ultrafiltration on membrane with a molecular weight cutoff (NMCWO) of from 500 to 50000 daltons, e.g. of 10.000 daltons, 5.000 daltons, 2500 daltons, 1000 daltons. The permeate fraction of extract thus obtained is substantially free from total apigenins and can be used as is or subjected to a lyophilisation process to provide a lyophilised fraction of extract useful for formulations having therapeutic employ for internal and/or external use.

According to the invention, therefore, said one or more ultrafiltration steps could be performed on a membrane having a cutoff of about 500-50,000 daltons, e.g. about 10,000 daltons, 5000 daltons, 2500 daltons, 1000 daltons.

The present invention therefore relates to a process for the preparation of fractions of chamomile extract depleted in total apigenins and/or depleted in essential oil, comprising the following steps:

a. preparing a hydroalcoholic extract from chamomile flowers and subjecting said extract to centrifugation and/or decantation, thereby obtaining a precipitated fraction A and a clarified alcoholic extract and collecting said precipitated fraction,
b. obtaining from the clarified alcoholic extract prepared in a. a concentrated aqueous extract,
c. decanting and/or filtering said aqueous extract, thereby collecting a water-soluble fraction (supernatant or filtrate),
e. subjecting the water-soluble fraction obtained in c. (supernatant or filtrate obtained in c) to one or more ultrafiltration steps and the permeate (fraction E) is recovered; wherein said permeate comprises total apigenins at a concentration of between 0.2% and 0.09% and a chamomile essential oil concentration of ≤0.01% expressed as weight%.

According to a further alternative embodiment the process of the invention may comprise, alternatively to the above-described step e., a step herein defined as step d. wherein the filtrate or the supernatant obtained in c. is subjected to one or more passages through a high-porosity adsorbing resin and the eluate is collected, wherein said eluate (fraction D) thus obtained comprises total apigenins at a concentration of ≤0.001% and a chamomile essential oil concentration of ≤0.01%, expressed as weight%.
In particular, the resin will be a resin able to adsorb aromatic and/or apolar substances, like, e.g., a high-porosity hydrophobic adsorbing resin. This type of resin consists of microspheres of a diameter of 0.2 mm - 0.08 mm, with an uniformity coefficient of ≤ 1.5 obtained by polymerization of Styrene and DVB without active groups, characterised by a highly porous physical structure having the parameter relative to the pore volume equal to about 1.3 ml/g enabling adsorption and selective elution of organic substances, preferentially of aromatic nature. A non-binding example of resin useful for the carrying out of the process, e.g., consists of amberlite XAD-2, serdolit PAD-II, ADS TQ 318 or resins similar thereto.
The present invention therefore also relates to a process for the preparation of fractions of chamomile extract depleted in total apigenins and chamomile essential oil comprising the following steps:

a. preparing a hydroalcoholic extract from chamomile flowers and subjecting said extract to centrifugation and/or decantation, thereby obtaining a precipitated fraction A and a clarified alcoholic extract and collecting said precipitated fraction,
b. obtaining from the clarified alcoholic extract prepared in a. a concentrated aqueous extract
c. decanting and/or filtering said aqueous extract, thereby collecting a water-soluble fraction (the supernatant or the filtrate);
d. said water-soluble fraction obtained in c. (the filtrate or the supernatant obtained in c.) is subjected to one or more steps (passages) through a hydrophobic adsorbing resin and the eluate (fraction D) is collected

wherein said eluate thus obtained comprises total apigenins at a concentration of ≤0.001% expressed as weight% and a concentration of chamomile essential oil of ≤0.01% expressed as weight%.
The fraction thus obtained is substantially free from total apigenins and can be used tel quel or subjected to lyophilization process to provide a lyophilised fraction of extract useful for formulations having therapeutic employ for internal and/or external use. According to the present invention, step d. can be carried out on a column with resins as described above, able to adsorb aromatic or highly apolar substances letting elute non-aromatic polar substances. The resin-adsorbed material, if desired, could be desorbed with a suitable solvent, like, e.g., ethanol or alcoholic solvents, for other uses.
Suitable chromatography resins may be, e.g., high-porosity adsorption resins of styrene-divinyl benzene copolymer, like, e.g., amberlite XAD-2, serdolit PAD-II or the like. Therefore the passage through resins having adsorbing features of the above-described type enables to obtain a fraction depleted in total apigenins (≤0.001 %).
The extract obtained with the above-indicated processes will contain only traces also of the other substances having a phenolic structure present in the initial product, besides total apigenins.
The process according to the present invention therefore enables to obtain fractions of chamomile extract as described above, depleted in total apigenins and essential oil and surprisingly rich in substances having a mucoadhesive action.
Moreover, the above-described process can eliminate also the resins by means of the reagents and steps used, as apparent to the technician in the field.
Therefore, in the case of fractions D and E, the fractions of the invention will also be substantially free from resins and essential oil, that will be found in the discarded fractions.
The present invention also relates to a composition comprising one or more fractions of chamomile extract useful in the protection of the skin or mucous membranes.
Such a protection, of course with reference both to the fractions (A, D, E, AD, AE, DE) per se or mixed with each other and to the composition, could be a preventive protection or a protection of partially compromised skin or mucous membranes as already described above.
For instance, the composition or the fractions can, by virtue of their mucoadhesion effect, facilitate the repair

of damaged skin or mucosal tissue, with entailed restoration of a healthy and elastic skin or mucous membrane.

The skin lesions according to the present invention may be, in case no cicatrizing and/or antimicrobial active principles be associated with the fractions of the invention, lesions that may imply also tissue underlying the skin or the mucous membrane and in which no open wounds are present.

By skin or mucosal lesions not implying the presence of open wounds, according to the present description, are meant those lesions in which the superficial layer of the skin, and the underlying layers, though not being wounded, are particularly fragile, irritated and damaged.

Non-limiting examples of this type of lesions are represented by first-degree burns, first-degree decubitus lesions, pressure lesions, newly-cicatrized rashes, irritations, erhytemas.

Optionally, in association with active ingredients suitable for the treatment of skin or mucosal lesions with open wounds, or in simultaneous or sequential administration with said active principles, the fractions of the invention could be as adjuvants of such treatment thanks to their mucoadhesion effect.

The compositions or the fractions (or mixtures thereof) of the invention can then be used for the treatment or the prevention of skin lesions not implying the presence of open wounds or in the prevention or slowing down of worsenings of the same or, as adjuvants, in the treatment of skin or mucosal lesions with open wounds.

The compositions according to the invention can advantageously comprise further components of plant and/or natural origin, having, e.g., emollient, digestive, prokinetics, cholagogue, carminative, prebiotic, relaxing, excipient, preservative, humectant properties, wherein said further components of plant origin are not total apigenins and/or chamomile essential oil.

Moreover, active ingredients of plant and/or natural origin, different from apigenins and/or from any active ingredients present in chamomile essential oil, could be added to the compositions of the invention.

In any case, therefore, the compositions of the invention could not have a concentration of apigenins, expressed as percentage by weight, greater than the concentration obtainable by using fractions A, D or E. Moreover, such compositions could also have a concentration of chamomile essential oil not greater than that obtainable by using fractions D or E.

Therefore, the compositions in all of the embodiments taught by the present description could have a concentration of total apigenins and of chamomile essential oil equal to that obtainable by using, for the preparation of such compositions, fractions A, D and/or E. In other words, to the compositions of the invention, total apigenins or chamomile oil could not be added to fractions A, B and/or E in the preparation of said compositions.

When the composition or one or more of the fractions of the invention are used for the protection of the skin, the application thereof will be topical. The embodiments of the compositions for topical use are reported hereinafter in the description.

The composition according to the invention could be made, e.g., as oil-in-water emulsion, water-in-oil emulsion, oil-in-gel emulsion or gel-in-oil emulsion, multiple emulsions, sprays, and anhydrous formulations (pomade, gel, paste, cream, ointment) and will comprise one or more excipients suitable for the making of the desired end form.

Such excipients could be, e.g., emulsifying agents (cetearyl alcohol, cetearyl glucoside, hydrogenated castor oil), rheological additives, antioxidants (like, e.g., vitamins, tocopherols or other antioxidants known in the field).

The emulsifying agent could be a surfactant, which by lowering the interfacial tension decreases the free energy of the system; alternatively, also non-surfactant substances can be used, such as gum arabic, gelatine, hydrophilic colloids or finely subdivided powders (e.g., talc). In one embodiment, the excipients could be present at an overall concentration in weight of between 3 and 8%, such concentration being of course indicative, taking into account that anyhow the technician in the field will know how to adapt the concentration of the necessary excipients according to the embodiment he/she intends to prepare without additional inventive activity.

Moreover, the composition could comprise perfuming and/or colouring agents making is odour pleasant, like, e.g., one or more essential oils like, e.g., lavender essential oil, melaleuca (tea tree), lemon, mint, orange essential oil, and/or colouring agents enabling, e.g., to easily recognize the zones of application of the composition, the colouring being, e.g., temporary so as not to interfere with other, subsequent applications of the composition.

In one embodiment, said colouring and/or perfuming agents are comprised at an overall concentration in weight between 0.001 and 3%.

By "overall concentration in weight" it is meant the concentration in weight in the composition of the sum of the various excipients, or the concentration in weight in the composition of the sum of the various perfuming and/or colouring agents present in the composition.

As to the use of the composition in the protection of the skin, the invention also relates to medical devices like, e.g., medicated patches, medicated gauzes, medicated bandages, medicated towels, medicated pads, medicated diapers, that is patches, gauzes, bandages, towels, pads or diapers which comprise, or are at least partially covered by or are at least partially imbued with the composition of the invention described in the most appropriate form.

The gauzes may be designed as grease gauzes, and similarly the bandages and patches using the composition designed in the form of an ointment, paste or cream, for example. The towels could be imbued with the composition in

the form of an oil emulsion with water or gel, and the diapers or the adsorbent pads could be made by inserting the composition in the relevant layers known in the art known in the art for the insertion of protective or anti-irritating compositions.

Such products such as infant diapers, adsorbent pads or common "sanitary pads" for women and adult incontinence pads, are commonly used for example in infancy- women- and geriatrics-related fields, and the person skilled in the art will know where to insert the composition described herein and which embodiment is the most suitable without the need of particular teachings and only based on the conventional techniques in the art.

Such devices will be applicable to the area to be treated and/or protected for a preventive purpose.

As already indicated above, the composition of the invention may be a composition for topical use which could be designed in the form of an oil-in-water emulsion, water-in-oil emulsion, multiple emulsions, spray, and anhydrous formulations (pomade, ointment, gel, paste, cream, spray) according to techniques commonly used in the art. The formulation herein named "spray" could be an anhydrous formulation or also a formulation in emulsion, the form with spraying dispenser also allowing self-applications in areas which are more difficult to reach (such as for example the back), useful in all those cases wherein the formulation is used, for example, to alleviate sun reddening, erythema or skin irritations of various kinds.

According to another embodiment, one or more of the fractions of the invention or the composition of the invention could be used, thanks to their mucoadhesive effect, for the protection of mucous membranes.

In this case as well, such protection could be a preventive protection, e.g. in all those cases envisaging an administration of drugs having the side effects of attacking the mucous membranes, or in those patients exhibiting conditions of recurring irritation of the same. In other cases, the protection could instead be a protection for curative purposes or in order to avoid the worsening of irritated or partially compromised mucous membranes.

In these cases, the administration of one or more of the fractions A, D, E or of the composition could be topical for all those mucous membranes on which a topical administration is possible (e.g., oral, rectal, vaginal, nasal mucosa) or oral in the cases in which said membranes be, e.g., intestinal or gastric mucous membranes.

The compositions for the protection of the mucous membranes could therefore be made in the form of capsule, tablet, lozenge, granule, powder, syrup, elixir, hard gelatine, soft gelatine, suspension, emulsion, solution, suppository, cream, gel, spray, ointment, pomade, paste, oil-in-water emulsion, water-in-oil emulsion, oil-in-gel emulsion, gel-in-oil emulsion.

In case of formulations for topical use, the above indications related to the compositions for the protection of the skin could be followed, or syrups, rinses, sprays could be made, e.g. for the protection of the mucous membranes of the mouth, throat, nose.

For application on the rectal mucosa, suppositories or enemas or microenemas could be used with the excipients known to the technician in the field for the making of such compositions.

As already mentioned, the composition of the invention could be in the form of capsule, tablet, lozenge, hard gelatine, soft gelatine, granule, powder, syrup, elixir, suspension, emulsion. For oral administration the composition could be made in the form of daily unit dosages or of fractions of daily unit dosages (e.g. 2, 3, 4, 5, 6 or more capsules, tablets, lozenges granule or powder single-doses, or gelatines could be taken over the day, in accordance with the judgment of the treating doctor), and may contain conventional excipients including, e.g., binding agents, like gum arabic gelatine, sorbitol, gum tragacanth, and/or polyvinylpyrrolidone; fillers, like lactose, sugar, corn starch, rice starch, calcium phosphate, sorbitol and/or glycine; tableting lubricants, like magnesium stearate, talc, polyethylenglycol and/or silica; disintegrants, e.g. potato starch; and wetting agents like sodium laurylsulphate. The tablets can be coated according to methods well-known in the standard pharmaceutical practice.

The composition could also be made in a liquid or semi-liquid form, as a suspension, emulsion, solution for oral administration and could optionally contain natural aromatizing agents giving a palatable taste thereto.

The composition in the form of powder or granule could be pre-metered in suitable containers and ready for use, either by ingestion as such or to be resuspended in an appropriate liquid such as water, tea, etc. In this case as well, the composition could contain natural aromatizing agents giving a palatable taste thereto.

Evidently, all of the above-indicated excipients could be used in a pharmaceutically acceptable grade.

In one embodiment, the composition as described herein, in any one of the above-indicated embodiments, could be in the form of pharmaceutical composition, i.e. comprise pharmaceutical-grade ingredients, or it could be or be introduced into a medical food or into a medical device.

The composition according to the present description could be made in the form of pharmaceutical composition or of medical device according to any one of the classes described in Directive 93/42/EEC on medical devices (comprising also substances and not only "devices" in the mechanical sense of the term), or in the form of medical food, food supplement, or in any form according to the regulatory provisions of the Country in which said composition will be produced.

The medical device or the medical food could also contain as ingredients other components, comprising e.g. combinations of vitamins, mineral salts and other substances directed at diet supplementing.

One or more of the fractions of the invention or the composition of the invention will therefore be useful for their barrier effect properties and, when present, mucoadhesion properties, in all those cases in which the protection of skin or mucous membranes is needed or desirable, which may be cases wherein a drug which attacks the mucous membranes or the skin has to be administered, therefore in order to prevent or limit the damaging action of the drug, or in those cases in which the protection of a partially compromised skin or mucous membrane is preferable or desirable so as to enable a better and quicker healing thereof, defending it from further aggressions, or in those cases in which an individual has a chronic disorder in which skin or mucous membranes undergo irritations or alterations whereby a barrier effect can prevent or limit damages on the skin or mucous membrane.

The invention also relates to a process for the treatment or for the prevention of the onset or the worsening of skin lesions not involving the presence of open wounds, or for the treatment of open wounds, wherein such process comprises one or more applications of the composition of the invention or of the medical device comprising it once or more per day on the concerned part.

The application of the composition, for instance, could be repeated whenever the need arises (e.g. at each change of pad in case of prevention of incontinence-related rashes) or once, twice, thrice, four or more times per day in general.

The invention also relates to a method for the preparation of a composition according to any one of the embodiments described above wherein fractions of chamomile extract depleted in total apigenins, and optionally in chamomile essential oil, as described herein (fractions A, D, E) or mixtures thereof are mixed with at least one of excipients and/or substances of natural and/or plant origin having emollient, digestive, pro-kinetics, cholagogue, carminative, prebiotic, relaxing, excipient, preservative, humectant properties as described above, with the above-indicated limitations.

Among these, e.g., could be used one or more of: extract of gentian root, boldo leaves extract, milk thistle fruits extract, artichoke leaves extract, dandelion root extract, anise fruit extract, rosemary leaf extract, mint leaves extract, marjoram leaves extract, cumin fruit extract, coriander fruit extract, ginger root extract, fennel fruit extract, caraway fruit extract, althea root extract, linseed extract, barleycorn extract, aloe leaves gel, charcoal, inulin.

In all of the above-indicated embodiments of the invention, the apigenin present in the compositions is only the apigenin present in fractions A, D and/or E.

Moreover, in all of the above-indicated embodiments the chamomile essential oil could be limited to be only that present in fractions A, D and/or E.

Object of the present invention is also a process for the protective (preventive or curative) treatment of the skin or the mucous membranes, providing the administration of one or more of the fractions or the composition of the present invention to a patient in need thereof. Such administration could also be concomitantly with the administration of other drugs.

The absence of pharmacologically active ingredients of chamomile in the fractions or in the composition makes such products particularly suitable to administration concomitantly with other drugs, as a side effect of interaction between the extract or the composition and the drug coadministered or concomitantly administered is markedly unlikely.

A non-limiting example of the process of treatment and/or of prevention of the skin or the mucous membranes could comprise the administration of a daily dosage, subdivided into a single dose or plural doses, described of the mixture or composition according to the present description, for a period of time of from one and six weeks, e.g. of from three and six weeks, or even for a period of time higher than six weeks, according to the judgment of the doctor in charge.

Such administration could precede the administration of the drug even for a prolonged period, so as to optimize the health state of the skin or of the mucous membrane to be treated.

The doctor in charge will know how to establish both the most appropriate dosage and the administration times, also on the basis of the patient's health state, weight, gender and age.

One of the substantial differences between the structure of the skin and that of the mucous membranes is represented by the absence, in the mucous membranes, of a selective barrier such as the corneous layer. Oral mucous membranes contact with noxious or irritating substances present in the environment (pollutants, pathogenic microorganisms, etc.) can therefore cause a high penetration of said substances both inside the mucous membranes and in the related airways (bronchi, lungs, etc.) causing inflammatory and/or allergic pathologies.

The protective effect of the extract of the present invention was assessed by mucoadhesion assay.

The cell adhesion assay performed with the fractions of the invention aims at assessing the mucoadhesivity of the product under examination in order to establish whether such product has the ability to adhere to the mucous membranes and consequently exert a protective action.

This enables to evaluate the mucoadhesive ability of the sample by inhibition of the lectin/glycoprotein bond expressed on the cell membrane, of cells coming from a mucous membrane.

**Evaluation of the mucoadhesive ability of the extract of the invention to mucosal cells, by evaluation of the percentage of inhibition of the lectin/glycoprotein bond**

In the first model, mucoadhesivity is determined by evaluation of the percentage of inhibition of the lectin/glycoprotein bond. In this model, for instance, buccal (oral), gastric, intestinal or vaginal cells may be used. Cells are initially treated with biotinylated lectin (Con-A), having high affinity for the glucoside and mannoside residues present in the glycoproteins of the membrane. The sites of the glycoproteins of the mucous membranes will thus be occupied with the biotinylated lectin. The presence of the biotin (vitamin H) in the lectin is indispensable for the next stage. The cells already treated with biotinylated lectin are charged with streptavidin peroxidase, making it possible to form the protein/glucose/lectin/biotin/ streptavidin peroxidase complex. At this point, the cells are washed and the protein/glucose/lectin/biotin/streptavidin peroxidase complex is quantified, thanks to the presence of the peroxidase, by means of a reaction of oxidation of the ortho-phenylenediamine. The protein/glucose/lectin/biotin/ streptavidin peroxidase complex catalyses the polymerization reaction:

$$\text{O-phenylenediamine} \xrightarrow[\text{Strep.perox}]{H_2O_2} \text{2,3-diaminophenazine (yellow).}$$

The intensity of the yellow/orange coloration of the solution (measured using a spectrophotometer with $\lambda$=450 nm) is proportional to the quantity of glycoprotein/lectin bonds and therefore to the quantity of available sites (glycoproteins) for mucoadhesion. The absorbency value thus determined constitutes the "control".

In the mucoadhesion assay reported below, the mucosal cells are treated for about 15 minutes at 30°C with the product under examination before the treatment with lectin. In the presence of mucoadhesive products, said products will inhibit lectin bonding, decreasing, proportionally to their mucoadhesion ability, the signal strength in the sample compared to the control as described above.

The percentage of mucoadhesion of the product (%MA) could be determined as

$$\%MA = (1 - \text{abs sample/abs control}) \times 100$$

## EXAMPLES

### Example 1

**Preparation of fractions A, D and E**

Chamomile flower heads as defined above were fragmented and subjected to two steps of extraction in decreasing concentration of ethanol, performing a step with 96% ethanol, and a step with 5% ethanol.

The alcoholic extracts obtained as described were gathered in a 50:50 ratio and subjected to centrifugation for a time of 1-5 minutes at a rotation rate equal to 3500 rpm; the precipitate constituting fraction A and the supernatant were recovered, and concentrated by ethanol evaporation with use of a thin film distillation system according to the standard protocol, providing the feeding of the extract to be concentrated at a rate of about 500 l/h; operation was by setting a residual vacuum of 0.6-0.8bars and the fluid heating the evaporation walls was set at 140°C, thereby obtaining, after ethanol elimination, a concentrated aqueous extract and a precipitate (fraction A).
Fraction A is characterised by a concentration of total apigenins of ≤ 0.08% by weight and essential oil of ≤0.4%
The aqueous extract thus obtained was centrifuged and/or filtered on panel filter with a cutoff of 25 microns.
The filtrate thus obtained was subjected to one or more steps of ultrafiltration on membrane with a cutoff of 2,500 daltons and/or on two membranes (10,000 and 2,500 daltons) in combination,

The permeate thus obtained (Fraction E) comprised total apigenins at a concentration in weight of ≤ 0.2 and essential oil at a concentration in weight of ≤ 0.01%.

The concentrated aqueous extract after settling and recovery of the clarified supernatant, or after filtration and recovery of the filtrate, was subjected, alternatively to the above-described ultrafiltration process, to a treatment with a high-porosity adsorbing resin, in particular a styrene and DVB copolymer resin of ADS TQ 318 type was used; evidently, any adsorbing resin with similar features could be used in the carrying out of the present invention.

The remaining active principles are retained by the resin and the eluate is collected.

By this process a fraction (fraction D) comprising total apigenins at a concentration in weight not greater than 0.001% and essential oil not greater than 0.01% is obtained.

In this case, a standard process for obtainment of the extract by treatment on resin entails the introduction of the concentrated aqueous extract in a chromatographic column containing the adsorption resin. The feed fluid must have suspended solids indicatively comprised between 0.2 at 10 Brix, the feed range is between 1 and 4BV/hour and preferably 2 BV/hour. The corresponding aqueous eluate is collected and subjected to drying by means of lyophilisation or desiccation. The substances adsorbed into the resin are eluated using 96% ethyl alcohol or methanol or acetone, preferably 96% ethyl alcohol. In this latter case, the alcohol eluate is subjected to desiccation or to lyophilisation (after having added in this latter case water at a ratio of 1:1 v/v with alcohol eluate and having evaporated the ethyl alcohol), and is useful for other purposes.

**Example 2**

**Evaluation of the mucoadhesive ability of the extract of the invention to mucosal cells, by evaluation of the percentage of inhibition of lectin-glycoprotein bonding**

The method described hereinafter derives from the optimisation of previous experimental protocols and scientific works on the topic (1-18), the mucoadhesivity of products intended for treatment of mucous membranes can be determined by evaluation of the percentage of inhibition of lectin/glycoprotein bonding. Mucosal cells of different origin (buccal, vaginal, gastrointestinal, etc.) can be used in this model. The assessment of mucoadhesive ability of chamomile fractions was carried out by using a HT-29 cell line (number ATCC HTB 38). The intestinal cells of the HT-29 line were selected as a model of epithelial cells, since they have morphological and biochemical characteristics typical of the absorbing enterocyte present in the gastrointestinal tract and have been widely used to study the functionality of the gastrointestinal cells and also to assess the transport and/or interaction of drugs through the gastrointestinal membrane (19-21). The bonding of the substance was assessed depending on the percentage of lectin-glycoprotein inhibition. Lectin is a protein contained in some leguminosae *(Canavalia ensiformis),* having a high affinity for the glucoside and mannoside residues present in the glycoproteins of the membrane. Lectin was artificially bonded to biotin (Con-A), which was able to bond to the streptavidin peroxidase that was added to the cellular suspension. Thus, the sites of the glycoproteins of the mucous membranes would be occupied with the biotinylated lectin. After a washing, o-phenylenediamine (o-pd) was added to the cell suspension in the presence of hydrogen peroxide. If in the reaction environment streptavidin peroxidase is present, o-pd is oxidised, with development of a yellow colour. The reaction was stopped after 2 minutes with $H_2SO_4$ 1 N. The intensity of the yellow/orange colouration of the solution (measured using a spectrophotometer with $\lambda$=492 nm) is proportional to the quantity of bonds of lectin with the glucoside groups on the cell membrane. In case of cells treated preliminarily with a product having mucoadhesive ability (incubation at 30°C for 15 minutes, before the treatment with lectin), the sites for bonding of lectin are occupied by the substance under examination, determining a decrease in the intensity of the end colouration, since less streptavidin peroxidase will be bonded to the biotinylated lectin. The decrease in the absorbency value will be proportional to the ability of substances under examination to "mucoadhere" to the cells. The mucoadhesive ability is expressed as percentage of inhibition of lectin-glycoprotein bonding, or better as percentage of mucoadhesion of the product according to Equation:

$$\text{Percentage of mucoadhesion of the product} = (1 - \text{abs sample/abs control}) \times 100$$

**Materials and methods**

Materials and methods

The HT-29 cells were kept in Mc Coy's with the addition of 10% of foetal bovine serum, 100 U/ml of penicillin and 100 $\mu$g/ml of streptomycin and held at 37°C in an incubator under humidified atmosphere formed of 95% air and 5% $CO_2$. The culture medium was changed every 2-3 days. Before the experiment, the cells were trypsinized, counted in a haemocytometer, centrifuged at 1000 rpm for 10 minutes and resuspended in 2 ml of culture medium. Then, the cells (contained in a total volume of 1 ml), subdivided into as many 50ml conical-bottom test tubes as the samples were, were treated with the solutions containing the sample or with the sole vehicle (BLANK: 5 ml of physiological solution). The solution containing the sample, contacted with the cell suspension, has an end volume of 5 ml; said volume is constituted 50:50 by physiological solution and solution containing the sample, in case of samples dissolved in hydroalcoholic solutions. In particular, the Inventors' samples were dissolved as follows: 60% hydroalcoholic solution in case of the reference extract, 20% hydroalcoholic solution in case of fraction C, physiological solution in case of fraction D. Similar responses were obtained in case of sample solubilisation in physiological solution. The conical-bottom test tubes were left under gentle stirring at 30°C for 15 minutes. The cells were then washed 3 times in TBS 0.05 M. 5 ml of TBS

0.05M containing $CaCl_2$ 1mM and 10mg/L of biotinylated lectin were then added, for 30 minutes at 30°C under slow stirring. After 1 wash, the cellular suspension was incubated with 5 ml of TBS 0.125M containing 5mg/L of streptavidin peroxidase and left to incubate for 60 minutes at 30°C under slow stirring. The cells were then washed 1 time and lastly resuspended in 1 ml of solution of o-pd (containing 0.4 mg of o-pd in citrate buffer and 0.4 μl of $H_2O_2$).
The oxidation of the o-pd produced a yellow colouration and the reaction was stopped after 2 minutes by the addition of $H_2SO_4$ 1N. The optical density was measured at 492 nm by means of spectrophotometric reading.

Table 2

| Fraction | Mucoadhesion (cell adhesion inhibition method) % |
|---|---|
| CHAMOMILE REFERENCE EXTRACT, LYOPHILISED | 63 |
| CHAMOMILE, FRACTION A, LYOPHILISED | 69 |
| CHAMOMILE, FRACTION D, RESIN-ELUTED FRACTION, LYOPHILISED | 30 |
| CHAMOMILE, FRACTION E, PERMEATE GH<2500 DA, LYOPHILISED | 40 |

The results obtained demonstrate that the assayed fractions of extract possess a high mucoadhesive ability with respect to mucosal cells. In light of the results obtained, it is possible to state that fraction A shows to possess a mucoadhesivity comparable to or greater than that of the reference lyophilised extract, whereas fractions D and E keep anyhow appreciable mucoadhesive abilities.

**Example 4**

**QUANTITATIVE DETERMINATION OF TOTAL APIGENINS IN HPLC**

Preparation of analysis solution. 0.50g ±0.025g of sample were weighed and extracted for 30min with 50ml of 40% EtOH by ultrasounds at the temperature of 35°C ±3°C. The obtained extract was centrifuged and decanted in a 100ml volumetric flask. The same extraction was repeated on the obtained residue. The extract obtained from the residue was centrifuged and decanted in the same 100ml volumetric flask. The obtained samples were brought to a volume of 100ml with the same extraction solvent at room temperature.
The 100 ml obtained were filtered with a 0.45μm cellulose acetate filter and injected in HPLC system
Chromatographic conditions:

Column: LC-18, 5μ + 4.6x250mm
column temperature: 20°C
detector: UV-Vis/DAD, λ= 335nm
flow: 1ml/min

mobile phase:

A = ultrapure $H_20$ + 0.2 %$H_3PO_4$
B = $CH_3CN$
C = MeOH

Table 3

| *min.* | *A %* | *B %* | *C %* | *ml/min* |
|---|---|---|---|---|
| 0 | 90 | 10 | 0 | 1 |
| 20 | 80 | 20 | 0 | 1 |
| 20.1 | 80 | 20 | 0 | 0.8 |
| 32 | 75 | 20 | 5 | 0.8 |

(continued)

| *min.* | *A %* | *B %* | *C %* | *ml/min* |
|---|---|---|---|---|
| 32.1 | 75 | 20 | 5 | 1 |
| 40.1 | 70 | 25 | 5 | 1 |
| 40.2 | 70 | 25 | 5 | 0.8 |
| 50 | 65 | 25 | 10 | 0.8 |
| 50.1 | 65 | 25 | 10 | 1 |
| 62 | 62 | 28 | 10 | 1 |
| 62.1 | 0 | 60 | 40 | 1 |
| 67 | 0 | 60 | 40 | 1 |
| 70 | 90 | 10 | 0 | 1 |
| Standard: Apigenin-7-O-glucoside<br>Solvent for standard dilutions: 40% methanol for HPLC | | | | |

**Example 5**

**QUANTITATIVE DETERMINATION OF ESSENTIAL OIL BY GC-MS**

**Preparation of the essential oil REFERENCE STANDARD**

100 g chamomile dried flower heads were introduced in the distillation flask of the apparatus for the distillation, under vapour stream, of essential oils. Then the essential oil was collected, diluted in hexane 1:1000, 1:2000 and 1:4000, and analyzed under the same instrumental conditions for sample analysis.

**Sample preparation.** 0.20g of sample were extracted with 10ml of Hexane for 30 minutes under ultrasounds (T°=35° C, W=52%, Hz=40). After centrifugation, on the pellet a second extraction is repeated under the same conditions. The solution brought to 20°C is brought to volume on a 20ml calibrated flask.

The solution thus obtained was filtered with a 0.45μm cellulose acetate filter.

<u>Chromatographic method</u>
Injector: 280°C, Splitless
Column HP-05MS with a length of 30m
Injection volume: 2μl
<u>Chromatographic conditions</u>
Carrier: Helium 0.9 l/min
Chromatographic ramp: 50°C until reaching 250°C, with a ramp of 6°C/min
<u>Acquisition method</u>
EI mass: 70eV.
Mode:SCAN
Masses: 50-300

**Result**

There were recognized the main constituents of essential oil, as reported in EP 7ed:

1. β-Farnesene, at 17.7 min
2. Bisabolol oxide B, at 21.7 min
3. α-Bisabolol, at 22.2 min
4. Bisabolone, at 22.28 min
5. Chamazulene, at 23.18 min
6. Bisabolol oxide A, at 23.4 min

In the samples, the areas corresponding to the 6 compounds were summed up and the percentage of essential

oil was evaluated by making reference to the sum of the same areas in the three dilutions.

**Example 6**

**Evaluation of mucoadhesion by adhesion assay with mucin on an inclined plane. Comparative test.**

Measurements are performed by an equipment comprised of a 45°-inclined plane, thermostated to 37°C, below said plane a microbalance is set, which performs measurements at regular 1-s intervals and prints recorded measurements on paper. Performed measurements are then transcribed and reprocessed on Excel spreadsheet.

The inclined plane acts as support for the biological substrate, consisting of a mucin film set up by depositing, on a plexiglas plane kept horizontal, a volume equal to 3ml of a suspension of porcine gastric mucin at an 8% concentration (w/w) in a phosphate buffer, pH 6.4.

The dispersion was brought to dryness at the temperature of 44°C so as to obtain a film of known surface area equal to 33.6 $cm^2$. Amount, concentration and deposition plane were defined during process development.

An exactly weighed amount of each analyzed fraction (i.e., fractions from flower heads according to the invention A, D and E and fraction from yellow tubular flowers, as described in Example 8 of document WO2009/138860, denoted as Fraction 4) was dissolved in solvent (ethanol 50°, subsequently diluted 1:2 in MEM Eagle-type medium with Earle's BSS; sample concentration: 10 mg/ml or 1%). The extract was deposited on the measurement plane with a multichannel pipette at constant time and rate during 6 seconds.

The multichannel pipette was adjusted so as to meter 250 microliters of solution (or dispersion) over the work plane. The total amount measured at each assay is of about 1 ml per sample (the weight of said volume is measured before the assay and depends on the density of the sample under analysis).

The sample, which would fall on the microbalance, was loaded in the top part of the inclined plane, containing the mucin film, and let slide thereon.

The amount of fallen sample was measured by recording the weight variation as a function of time.

The assay lasted 40 seconds, however it is stressed that after 20 seconds there was no weighing variation.

Measurements in the absence of the mucin film were also performed (measurements indicated as blank) with the same amounts of sample and under the same assaying conditions, but without the mucin layer deposited on the plane. Thus, it is possible to assess the intrinsic sliding properties of the sample, regardless of its ability to interact with the biological substrate.

The adhered % is calculated: defined as the amount of sample remained adhered to the mucin film or to the inclined plane (without mucin, blank) at the end of the measurement, normalized with respect to the amount deposited on the inclined plane.

It is determined the mucoadhesion ability in terms of percentage difference between sample amount adhered to the plane with mucin and sample amount adhered to the plane without mucin. The calculation is performed in accordance with the following equation:

$$\text{\% difference} = (\text{adhered mucin \%} - \text{adhered blank \%}) / \text{adhered \% of the blank.}$$

Where:

- adhered mucin %= sample percentage remained adhered to the mucin film at the end of the measurement
- adhered blank %= sample percentage at the inclined plane at the end of the blank measurements.

As mentioned above, fraction 4 was prepared as described in Example 8 of document WO2009/138860.

In Figure 2, data obtained from the experiment are reported. The experiment was performed in triplicate and gave the following average data:

Table 4

| | Adhered blank % (average values; n=3) | Adhered mucin % (average values; n=3) | PERCENTAGE DIFFERENCE % |
|---|---|---|---|
| Fraction A: | 11.80 | 22.20 | 88.14 |
| Fraction D: | 11.70 | 20.30 | 73.50 |
| Fraction E: | 7.70 | 20.60 | 167.53 |

(continued)

| | Adhered blank % (average values; n=3) | Adhered mucin % (average values; n=3) | PERCENTAGE DIFFERENCE % |
|---|---|---|---|
| Fraction 4: | 22.20 | 25.10 | 13.06 |

The data obtained show how the mucoadhesive properties of the fractions of the invention be remarkably greater than those of fractions of chamomile tubular flowers described in the literature.

## Claims

1. A fraction of chamomile flower heads extract depleted in total apigenins, wherein said total apigenins have a concentration expressed as percentage by weight of between 0.2% and 0.09%, ≤0.08% or ≤0.001%.

2. The fraction of extract according to claim 1 wherein said total apigenins have a concentration of between 0.2% and 0.09% expressed as weight% and a concentration of chamomile essential oil of ≤0.01% expressed as weight% or wherein said total apigenins have a concentration of ≤ 0.001% expressed as weight% and a concentration of chamomile essential oil of ≤0.01% expressed as weight%

3. The fractions of extract according to claims 1 or 2, wherein said fractions are hydrophilic fractions or mixed hydrophylic/lipophylic fractions.

4. The fractions of extract according to any one of claims 1 to 3 or mixtures thereof for use in a treatment for the protection of the skin or mucous membranes.

5. The fractions of extract according to claim 4, wherein said mucous membranes are the oral mucosa, gastric mucosa, intestinal mucosa, the nasal mucosa, the vaginal mucosa, the uterine mucosa, the rectal mucosa.

6. A process for the preparation of fractions of chamomile flower heads extract depleted in total apigenins comprising the following step:

   a. preparing a hydroalcoholic extract of chamomile flower heads and subjecting said extract to centrifugation and/or decantation, thereby obtaining a precipitated fraction A **characterised by** a concentration of total apigenins of ≤0.08% by weight and a clarified alcoholic extract, and collecting said precipitated fraction A.

7. The process according to claim 6, wherein said hydroalcoholic extract in step a. and said fraction A are prepared by subjecting chamomile flowers, optionally fragmented, to two or more extraction steps in decreasing concentration of ethanol.

8. The process according to any one of claims 6 or 7, further comprising the following steps:

   b. obtaining from the clarified alcoholic extract prepared in a. a concentrated aqueous extract,
   c. decanting and/or centrifuging and filtering said aqueous extract, thereby collecting a water-soluble fraction,
   d. subjecting the water-soluble fraction obtained in c. to a step on adsorbing resin, thereby collecting a fraction D, corresponding to the eluate obtained from said passage on resin, **characterised by** a total apigenins concentration of ≤0.001% and an essential oil concentration of ≤0.01%, expressed as weight%.

9. The process according to claim 8, wherein said passage on resin is carried out on a high-porosity adsorbing resin of styrene and DVB copolymer.

10. The process according to any one of claims 6 or 7, further comprising the following steps:

   b. obtaining from the clarified alcoholic extract prepared in a. a concentrated aqueous extract,
   c. decanting and/or centrifuging and filtering said aqueous extract, thereby collecting a water-soluble fraction;
   e. subjecting the water-soluble fraction obtained in c. to ultrafiltration on a membrane and collecting a fraction E, corresponding to the permeate obtained from said ultrafiltration, **characterised by** a total apigenins concentration of between 0.2% and 0.06% and a chamomile essential oil concentration of ≤0.01% expressed as weight%.

**11.** The process according to claim 10, wherein said one or more ultrafiltration step is carried out on a membrane having a cutoff of about 500 to 20,000 daltons.

**12.** A pharmaceutical carrier comprising one or more fractions according to any one of claims 1 to 5, wherein said carrier promotes the adhesion of the active ingredients to the skin or to the mucous membranes.

**13.** A composition comprising one or more fractions of chamomile extract according to any one of claims 1 to 5 wherein the sole apigenin present in said composition is the apigenin present in said one or more fractions.

**14.** A composition comprising one or more fractions of chamomile extract according to claim 13, wherein the sole essential oil of chamomile present in said composition is the essential oil present in said one or more fractions.

**15.** The composition according to claim 13 or 14 for use in the protection of the skin or mucous membranes.

**16.** The composition according to claim 15 wherein said mucous membranes are the oral mucosa, gastric mucosa, intestinal mucosa, the nasal mucosa, the vaginal mucosa, the uterine mucosa, the rectal mucosa.

**17.** The composition according to any one of claims 13 to 16, further comprising substances of natural and/or plant origin having emollient, digestive, pro-kinetics, cholagogue, carminative, prebiotic, relaxing, excipient, preservative, humectant properties.

**18.** The composition according to any one of claims 13 to 17, in the form of capsule, tablet, lozenge, granule, powder, syrup, elixir, hard gelatine, soft gelatine, suspension, emulsion, solution, suppository, cream, gel, spray, ointment, pomade, paste, oil-in-water emulsion, water-in-oil emulsion, oil-in-gel emulsion, gel-in-oil emulsion.

**19.** The composition according to any one of claims 13 to 18, wherein said composition is or is comprised in a pharmaceutical composition, is comprised in or consists of a medical device, or is comprised in a medical food.

**20.** A medical device, medicament, or a medical food comprising a composition according to any one of claims 13 to 18 or one or more fractions according to any one of claims 1 to 5.

**21.** A method for the preparation of a composition according to any one of claims 13 to 18 wherein one or more fractions of chamomile extract depleted in total apigenins selected from fractions **characterised by** a total apigenins concentration of between 0.2% and 0.09%; ≤0.08% and ≤0.001% of total apigenins is mixed with at least one of excipients and/or substances of natural and/or plant origin having emollient, digestive, pro-kinetics, cholagogue, carminative, prebiotic, relaxing, excipient, preservative, humectant properties.

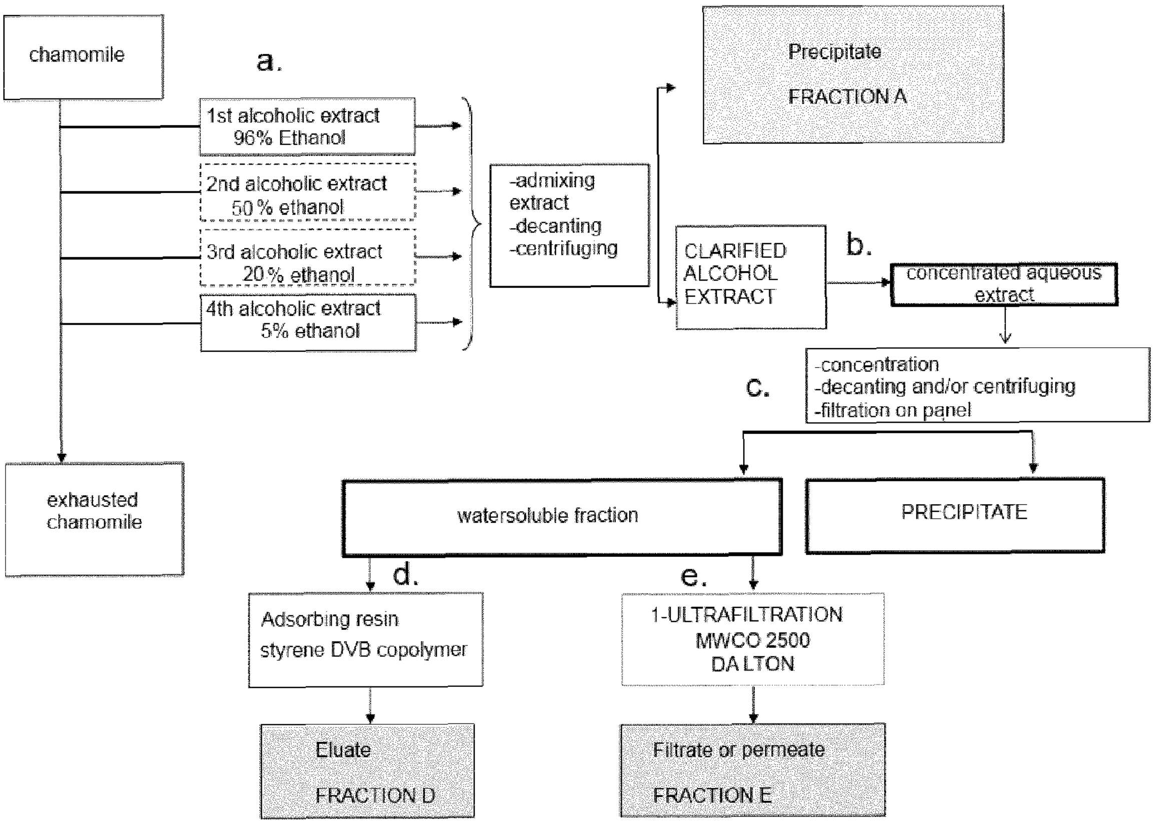
chamomile
a.
1st alcoholic extract
96% Ethanol
2nd alcoholic extract
50 % ethanol
3rd alcoholic extract
20 % ethanol
4th alcoholic extract
5% ethanol
-admixing
extract
-decanting
-centrifuging
Precipitate
FRACTION A
CLARIFIED
ALCOHOL
EXTRACT
b.
concentrated aqueous
extract
-concentration
-decanting and/or centrifuging
-filtration on panel
c.
exhausted
chamomile
watersoluble fraction
PRECIPITATE
d.
e.
Adsorbing resin
styrene DVB copolymer
1-ULTRAFILTRATION
MWCO 2500
DALTON
Eluate
FRACTION D
Filtrate or permeate
FRACTION E

Fig. 1

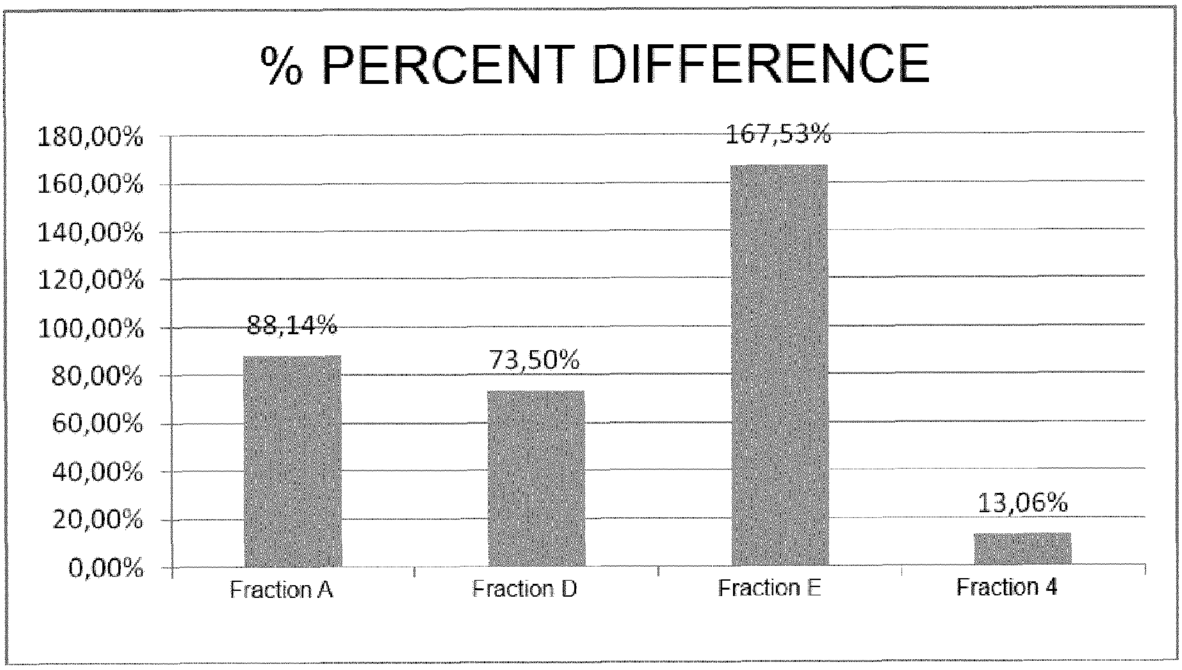
% PERCENT DIFFERENCE
180,00%
160,00%
140,00%
120,00%
100,00%
80,00%
60,00%
40,00%
20,00%
0,00%
88,14%
73,50%
167,53%
13,06%
Fraction A
Fraction D
Fraction E
Fraction 4

Fig. 2

Europäisches Patentamt
European Patent Office
Office européen des brevets

# EUROPEAN SEARCH REPORT

Application Number
EP 14 19 5927

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | EP 0 096 016 A2 (BONOMELLI SPA [IT]) 7 December 1983 (1983-12-07)<br>* the whole document *<br>* page 2, lines 20-34 *<br>----- | 1-6, 12-21<br>1-21 | INV. A61K36/28 |
| X<br><br><br>Y | US 2009/285921 A1 (KREUTER MATTHIAS-HEINRICH [CH]) 19 November 2009 (2009-11-19)<br>* the whole document *<br>* claims 1, 12, 14 *<br>----- | 1-5, 12-21<br><br>1-21 | |
| X<br><br><br>Y | WO 2009/138860 A1 (VERITRON LTD [GB]; KREUTER MATTHIAS-HEINRICH [CH]) 19 November 2009 (2009-11-19)<br>* the whole document *<br>* claims 1, 5, 6, 7 *<br>* examples 7-9, 14, 15, 16 *<br>----- | 1-6, 10-21<br><br>1-21 | |
| X<br><br><br>Y | WO 2013/118099 A1 (ABOCA SOCIETA S P A SOCIETA AGRICOLA [IT]) 15 August 2013 (2013-08-15)<br>* the whole document *<br>* claims 1-15 *<br>* page 12, lines 6-23 *<br>----- | 1-6, 12-21<br><br>1-21 | TECHNICAL FIELDS SEARCHED (IPC)<br>A61K |
| X<br><br><br><br><br><br><br><br><br>Y | FUELLER E ET AL: "Anti-inflammatory activity of Chamomile polysaccharides", PHARMACEUTICAL AND PHARMACOLOGICAL LETTERS, MEDPHARM, SCIENTIFIC PUBL., STUTTGART, DE, vol. 10, no. 2, 1 January 2000 (2000-01-01), pages 86-87, XP009103344, ISSN: 0939-9488<br>* the whole document *<br>----- | 1-5, 12-21<br><br><br><br><br><br><br><br><br>1-21 | |

The present search report has been drawn up for all claims

1

| Place of search | Date of completion of the search | Examiner |
|---|---|---|
| Munich | 26 March 2015 | Fayos, Cécile |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 19 5927

This annex lists the patent family members relating to the patent documents cited in the above-mentioned European search report. The members are as contained in the European Patent Office EDP file on
The European Patent Office is in no way liable for these particulars which are merely given for the purpose of information.

26-03-2015

| Patent document cited in search report | Publication date | Patent family member(s) | Publication date |
|---|---|---|---|
| EP 0096016 A2 | 07-12-1983 | DE 3371170 D1 | 04-06-1987 |
| | | EP 0096016 A2 | 07-12-1983 |
| | | IT 1157945 B | 18-02-1987 |
| | | US 4584198 A | 22-04-1986 |
| US 2009285921 A1 | 19-11-2009 | AU 2009247689 A1 | 19-11-2009 |
| | | CA 2723860 A1 | 19-11-2009 |
| | | CH 698908 A1 | 30-11-2009 |
| | | CN 102123719 A | 13-07-2011 |
| | | EP 2288363 A1 | 02-03-2011 |
| | | EP 2805725 A1 | 26-11-2014 |
| | | JP 5643949 B2 | 24-12-2014 |
| | | JP 2011520867 A | 21-07-2011 |
| | | KR 20110021893 A | 04-03-2011 |
| | | RU 2010151658 A | 27-06-2012 |
| | | TW 201006483 A | 16-02-2010 |
| | | US 2009285921 A1 | 19-11-2009 |
| | | US 2011212197 A1 | 01-09-2011 |
| WO 2009138860 A1 | 19-11-2009 | NONE | |
| WO 2013118099 A1 | 15-08-2013 | EP 2812013 A1 | 17-12-2014 |
| | | US 2015023948 A1 | 22-01-2015 |
| | | WO 2013118099 A1 | 15-08-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0096016 A
- US 2009285921 A
- WO 2009138860 A